# Europäisches Patentamt
# European Patent Office
# Office européen des brevets

(19)

(11) Publication number: **0 057 609**
**B1**

(12) **EUROPEAN PATENT SPECIFICATION**

(45) Date of publication of patent specification: **13.11.85**

(51) Int. Cl.⁴: **A 63 B 23/04**, A 61 H 1/02

(21) Application number: **82300509.5**

(22) Date of filing: **01.02.82**

(54) Exercising apparatus.

(30) Priority: **30.01.81 US 229922**

(43) Date of publication of application:
**11.08.82 Bulletin 82/32**

(45) Publication of the grant of the patent:
**13.11.85 Bulletin 85/46**

(84) Designated Contracting States:
**AT BE CH DE FR GB IT LI LU NL SE**

(56) References cited:
**GB-A- 925 678**
**GB-A-1 406 342**
**US-A-3 529 474**
**US-A-3 745 990**
**US-A-3 869 121**

(73) Proprietor: **NAUTILUS SPORTS/MEDICAL**
**INDUSTRIES, INC.**
**305 East Ohio Avenue**
**Lake Helen Florida 32744 (US)**

(72) Inventor: **Jones, Arthur Allen**
**Idle Hour Farm 1155 North East 77th Street**
**Ocala Florida (US)**

(74) Representative: **Warren, Keith Stanley et al**
**BARON & WARREN 18 South End Kensington**
**London W8 5BU (GB)**

Courier Press, Leamington Spa, England.

## Description

Planned programs of exercise have long been followed by persons training for athletic events of various types or pursuing a course of treatment for the rehabilitation of an illness, injury or the like. Many such planned programs of exercise have employed aids which have, in recent years, come to take the form of various apparatus or machines which facilitate the planned programs of exercise. Such apparatus, machines and methods have grown out of various concepts and studies regarding physiology and physiological development and function.

One type of apparatus which has been used with significant success is full range exercise such as is possible, for example, through use of the apparatus of Jones, United States patent 3,858,873. "Full range" exercise as used with reference to such an apparatus and method, and as used on the description which follows, is a defined term. The defined term "full range exercise" refers to exercise having positive work; negative work; rotary form movement; stretching; prestretching; automatically variable, balanced, direct resistance; resistance in the position of full muscular contraction; and unrestricted speed of movement. The interested reader is referred to available publications for further explication of these characteristic features of full range exercise.

It is recognized, however, that not all exercise need be full range exercise as here defined. More specifically, certain exercise movements are compound movements which cannot attain the characteristics of full range exercise as listed briefly above due to the presence of "lock out" positions and non-rotary movements. Such exercise may, for example, be realized through the use of apparatus such as that disclosed in Mazman United States patent 3,905,599.

The general field of such exercise and related apparatus and methods is here referred to as "exercise physiology." The field of exercise physiology is related to sports medicine, being the field of medical study which is particularly directed to athletic sports and the like. While it has become possible, within these fields, to develop exercise programs which apply methods and apparatus of the types briefly mentioned above with greater specificity than has been possible heretofore, exercise physiology and related areas of sports madicine have continued to be inherently imprecise. A major difficulty in accomplishing precise application of exercising programs has been in the difficulty of monitoring, directing and recording physiological performance.

One type of apparatus and method for attempting to monitor and record physiological performance is illustrated by Dimick in British Patent 1,406,342. Dimick discloses an exercise bicycle which allegedly can monitor and display certain characteristics of the bicycle's performance, and thus can potentially monitor and display, in a secondary fashion, the physiological performance of the exercising user.

In Dimick's device, the load against which the user must perform is provided by the apparatus, specifically by a flywheel frictionally angaged by a belt and by a cam mechanism which can apply a load to the belt. Portions of the flywheel belt pass over a movable pulley such that when the load on the belt is varied, as by greater exertion of the exercising user, the position of the pulley is also varied. A monitoring system measures the displacement of the pulley and consequently can give an indication of the force being applied thereto. Dimick's device cannot, however, monitor the force being exerted directly by the user, nor can it monitor user's position at any given moment in the exercising cycle. Finally, lacking those two direct measurements, Dimick's device cannot monitor or display the user's exertion at any time or position, but rather can only give an indication of the secondary force being applied to the movable pulley.

It is an object of the present invention to provide an apparatus having means such that during use, all of the resistance load provided against the exercising person is provided by the exercising person. In this regard, the rate of work is also governed by the exercising person. The apparatus thus exhibits a greater safety than conventional devices, in that no force is generated by the apparatus itself, and in that the device requires the user to develop coordination skills by consciously producing the required force and speed in exercising.

More particularly, the present invention provides exercise apparatus for providing a user with a range of exercise movements and recording a measure of the effort exerted, said apparatus including:

first body engaging means adapted to be contacted by a first portion of a user's body and to be moved therewith; and

second body engaging means adapted to be contacted by a second portion of a user's body and to be moved therewith;

interconnecting means linking said first and second body engaging means in a force transmitting passive relationship for effecting predetermined coordinated movement of said first and second body engaging means with respect to one another, said interconnecting means converting positive musculature work applied to one of said body engaging means to negative musculature work supplied to said other body engaging means, there being substantially no other force applied to and no resistive forces acting upon said body engaging means, whereby all forces and resistances are provided solely by the user and may be instantaneously varied by said user;

force-sensing means operatively connected to said interconnecting means and producing an output signal proportional to the magnitude of the force applied to at least one of said body engaging means;

display means responsive to said processing means output signal to generate a cognizable display;

characterized by:

means whereby all forces and resistances are provided solely by the user and may be varied by said user such that there are substantially no other forces applied to and no resistive forces acting upon said body engaging means;

position sensing means operatively connected to said interconnecting means and producing an output signal proportional to the position of at least one of said body engaging means; and

processing means responsive to said force sensing means and said position sensing means to provide an output signal proportional to the physical effort exerted by the user as represented by the applied forces and changes of position.

The apparatus embodying the invention has position sensing means, force sensing means, processing means and display means, all interrelated such that the display means will highlight to the user the output of the processing means, which in turn reflects the input of both the force sensing means and the position sensing means. In this manner the apparatus further enhances the development of the fitness level and coordination skills to which it is directed.

The accuracy and precision by which physiological performance is monitored, directed and recorded, is improved. In this respect, the apparatus is provided with an open loop system for monitoring the instantaneous physical effort exerted by the exercising person, monitoring the instantaneous position of the exercising person and for correlating the monitored efforts and positions to a programmed standard. The open loop system displays a representation of the correlation of the monitored effort exerted to the programmed standard, in such a manner as to direct the physiological performance of the exercising person.

Reference will now be made to the accompanying drawings, in which:

Figure 1 is a perspective view of a leg extension exercise apparatus embodying the present invention;

Figure 2 is a side elevation view of the exercise apparatus of Figure 1, with certain cover components removed in order to make certain operating elements of the apparatus more readily visible;

Figure 3 is a front elevation view of the apparatus of Figure 2;

Figure 4 is a view of a display incorporated in the apparatus of Figures 1 through 3, taken generally as indicated by the arrow 4 in Figure 2;

Figure 5 is a graphic representation of one form of data display available from the apparatus of Figures 1 through 3, in accordance with the present invention;

Figure 6 is a schematic representation of the interconnection of and communication among certain apparatus used in accordance with the methods of this invention;

Figure 7 is a simplified flow chart representing certain steps in the methods of this invention;

Figure 8 is a view similar to Figure 1 of a second type of exercise apparatus in accordance with the present invention;

Figure 9 is a view similar to Figure 2 of the exercise apparatus of Figure 8;

Figure 10 is a view similar to Figure 3 of the exercise apparatus of Figure 9; and

Figures 11 and 12 are enlarged elevation views showing certain details of the apparatus of Figures 8 through 10.

While the present invention will be described hereinafter with particular reference to the accompanying drawings, in which certain operating embodiments of the apparatus of the present invention are shown, it is to be understood at the outset of the description which follows that it is contemplated that the apparatus may be varied from the specific forms described hereinafter, while still attaining the desired result of this invention. Accordingly, the description which follows is to be understood as a broad teaching disclosure directed to persons of appropriate skill in the appropriate arts, and not as limiting upon the scope of this invention.

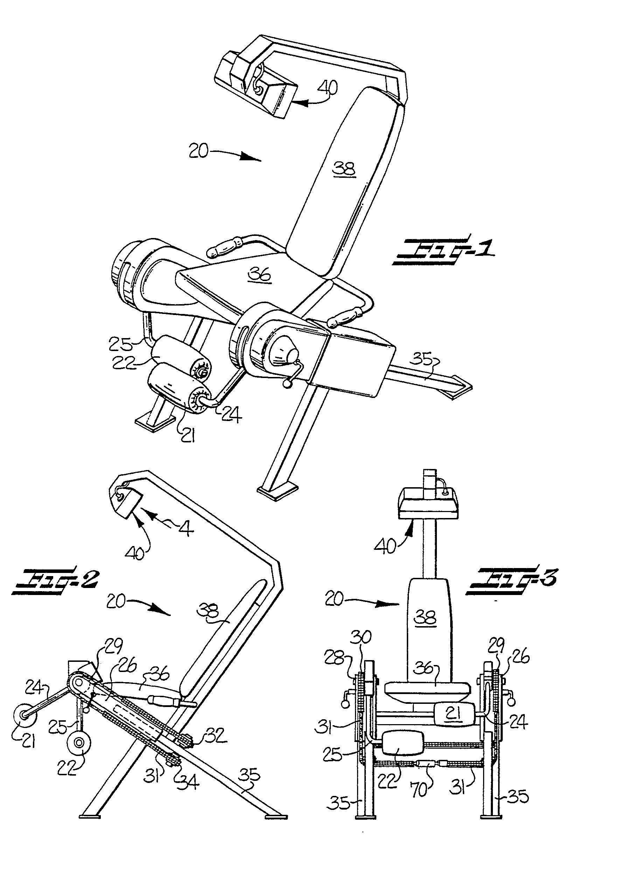

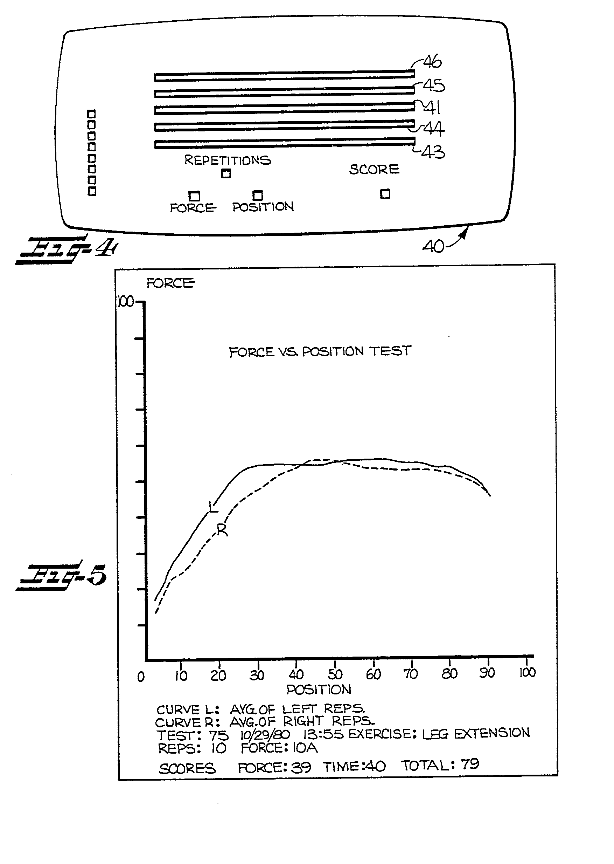

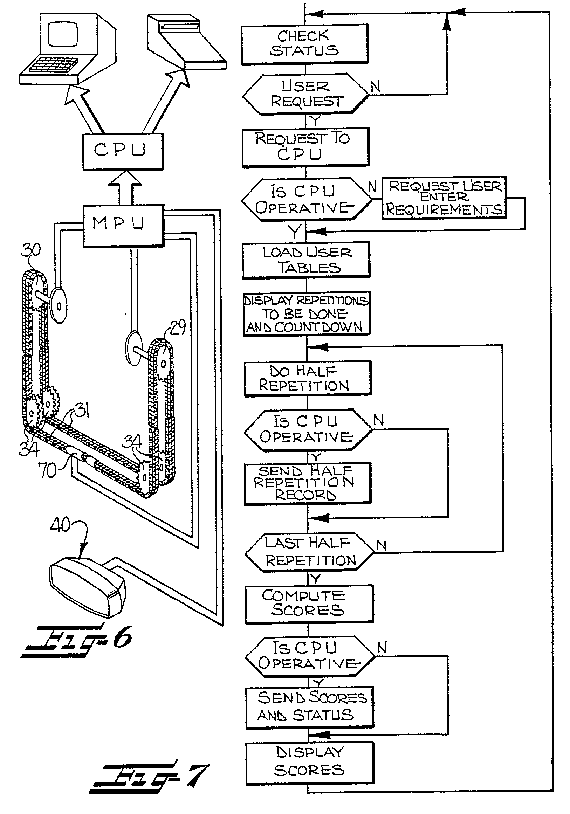

Referring now to Figures 1 through 3, an exercise apparatus embodying the present invention is there illustrated in the form of a leg extension apparatus generally indicated at 20. In the form illustrated, the apparatus 20 is a full range exercise apparatus having means engaged by body portions of an exercising person operating the apparatus. By means of mounting arrangements provided and operative coupling of elements with the body engaging means, the body engaging means rotates about an axis substantially coaxial with the axis of rotation of an engaged body portion of an exercising person and imposes throughout a full range of movement of the engaged body portion an automatically variable, balanced, direct resistance to the musculature effecting movement thereof. The imposition of resistance occurs while an unrestricted speed of movement is accommodated and so as to cause the musculature of the exercising person to perform both positive and negative work, and to be subjected to stretching and prestretching, all during rotary form movement of the engaged body portion. In the specific form illustrated, the leg extension apparatus 20 has first and second roll members 21, 22 respectively engaged by shin surfaces of the left and right legs of an exercising person operating the apparatus 20. Each of the first and second roll members 21, 22 is mounted for rotation about an axis, by means of arm members 24, 25. In use, the axes of rotation of the arm members 24, 25 and roll members 21, 22 moving therewith are substantially coaxial with the axis of rotation at the knees of the person operating the apparatus 20.

In the form of apparatus illustrated in Figures 1 through 3, means are provided which are operatively coupled with the first and second body engaging means 21, 22 for imposing throughout a full range of movement of the lower leg portions of the exercising person a resistance of the type described. In particular, each of the arm members 24, 25 is joined, near the axis of rotation thereof, with a stub shaft which extends through a side frame structure of the apparatus 20. The side frame structures include side frame members 26, 28. Affixed to the respective shafts are sprockets 29, 30 about which is trained a chain 31. The chain 31 is formed as an essentially closed loop, passes over rear sprockets such as the sprockets 32, 34 mounted on a rear leg member 35, and are arranged so that upward movement of one roll member 21 brings about downward movement of the other roll member 22, and vice versa. As will be understood, the positive work being done by one lower leg (for example, the left lower leg of an exercising person) provides, through the chain 31, the resistance which brings about negative work done by the other body portion (for example, the right leg). Thus, in the form of apparatus 20 illustrated in Figures 1 through 3, all resistance provided is provided by the exercising person. Further, the rate of work is governed by the exercising person. The apparatus 20 thus is both quite safe, in that no force is generated by the apparatus 20 itself, and requires the user to develop coordination skills in that the exercising person must consciously produce the required force and speed in exercising. These features relate to other characteristics of the present invention as described more fully hereinafter.

In using the apparatus of Figures 1 through 3, an exercising person assumes a position seated upon a seat portion 36, which is mounted on the frame of the apparatus 20, and leans backward against a backrest portion 38. The user's legs, dangling forwardly over the front edge of the seat 36, are positioned for substantially coaxial alignment of the knee joint and the rotational axes for the roll members 21, 22. As so positioned, the user is supported so as to be able to readily view a display generally indicated at 40 and described more fully hereinafter.

In accordance with important features of this invention, open loop means are operatively coupled with the body engaging means of the apparatus 20 for monitoring physical effort exerted by an exercising person operating the apparatus and for correlating monitored effort exerted to a programmed standard and for displaying, by means of the display generally indicated at 40, a representation of the correlation of monitored effort exerted to the programmed standard. It is significant to the present subject invention that the apparatus 20 includes an open loop means. As is familiar to persons skilled in the art of servomechanisms, a closed loop involves feeding back into an apparatus some signal derived from the action of the apparatus. In the specific instance of an exercise apparatus, such a "closed loop" might involve a servomechanism for exerting force with respect to an exercising person. Such servomechanisms have an inherent risk of injury, in that the servomechanism may become unstable or may fail in such a way as to inflict serious injury to the exercising person. That is not the case in the apparatus 20 of the present invention. In the apparatus 20 of the present invention, closure of the open loop, if any, occurs through the actions of the exercising person who, as briefly indicated above, maintains control over the force exerted, the speed of movement, and the consequent work done and physical effort exerted. In the form shown, this remains particularly true inasmuch as the sole source of force exerted is the exercising person. However, it is known that essentially the same characteristics follow where a weight stack or the like is used as a portion or all of the force exerting means. Use of a weight stack or the like simply presents an additional preset or predetermined factor to be taken into account in operation of the apparatus as described more fully hereinafter.

In the form contemplated for the apparatus 20 of Figure 1 through 3, the open loop system includes means operatively coupled with the roll members 21, 22 for responding to exertion of force between those body engaging means and the engaged shin portions and for signalling the magnitude of force exerted. Preferably, the force responsive and signalling means comprises a load cell inserted into the chain 31 as a link thereof and which develops an appropriate analogue electrical signal. One form of load cell which has been employed successfully in apparatus such as the apparatus 20 of Figures 1 through 3 is an electrical resistance strain gauge which varies in resistance as force is exerted thereon. Means are additionally operatively coupled with the body engaging roll members 21, 22 for responding to movement thereof and for signalling the position thereof. Preferably, the position responsive signalling means takes the form of a pair of potentiometers aligned with the shafts which carry the upper front sprockets 29, 30 and which define the axis of rotation for the roll members 21, 22. Potentiometers, used at this point, signal the position of the arms 24, 25 and the roll members 21, 22 by variations in the resistance value.

While it is recognized that the chaining together of the arms 24, 25 as described hereinabove, by means of the chain 31 which includes the load cell, essentially coordinates the movement of the two arms and the body engaging means carried thereby, opening the possibility of using a single position signalling means, it is preferred to provide two position signalling means as described above. The use of two signalling means reduces the possibility of introducing error due to chain stretch and the like.

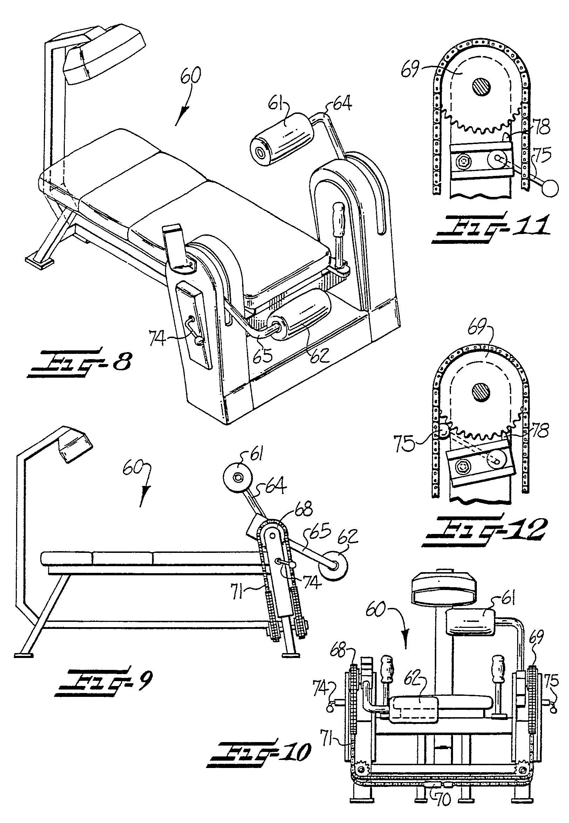

The load cell and the position signalling potentiometers are operatively connected with and supply signals to a monitoring and display means which includes the display visible to a user of the apparatus 20 in the housing 40 as briefly described above. The display, as visible to the user of the apparatus 20, is illustrated in Figure 4 and includes both analogue and digital information. The display means is operated under the control of digital processing means operatively connected with the analogue signalling means described hereinabove. The digital processing means preferably includes a microcomputer or processor forming an integral part of the apparatus 20. The digital processing means includes a stored program means for retaining instructions regarding at least one of a standard magnitude of force to be exerted by an exercising person and a standard rate of exertion of force by the exercising person and a standard number of repetitions of cyclical force exertion.

Use of a microprocessor or microcomputer as a component of the apparatus 20, as described hereinabove, provides a range of significant advantages for the apparatus of the present invention, particularly in an environment such as a laboratory, health club or spa in which a plurality of machines of various types may be present. The apparatus 20, with a microprocessor or microcomputer as a component thereof, is an "intelligent machine". That is, the machine can identify a particular user from a code entered manually or by means of a coded card or the like and can communicate with a central processing unit which monitors a plurality of apparatus for retrieving from a memory which forms a portion of the apparatus 20 or which forms a portion of the central processing unit a particular exercise regimen to be followed by a particular user. Further, as the exercise regimen is carried out, the force applied by the exercising person and the position from which force is applied are monitored continuously and the information derived from the monitoring is processed. As a consequence, the user while operating the apparatus 20 may view a display of the representation of the user's signalled effort and its correlation to the user's programmed regimen of exercise.

In the particular form of the display illustrated in Figure 4, a plurality of generally horizontal indicator lines are provided, respectively identified in Figure 4 at 41, and 43 through 46. Line 41 is used to display a progressive pacing signal which may, for example, take the form of a gradually extending lighted line moving from one side of the display toward the other at a timed rate. Such a display may be generated for example, by successively signalling a series of light emitting diodes. The other display lines 43 through 46 may have similar devices which may be successively signalled to provide an analogue display of a gradually extending lighted line. The display to be generated on the other display lines 43 through 46 is responsive to exercise effort exerted by the exercising person and thus will move from one side of the display toward the other at a rate dependent upon the rate of movement of the exercising person. Further the signal will move from one to another of the lines 43 through 46 in response to variations in force exerted. That is, an exercising movement performed at an indicated rate but with low levels of force will be displayed only on line 43. An exercising effort which involved exertion of varying levels of force might begin a trace on the lowermost line 43, move upward through lines 44 and 45 to line 46, and then return back toward the lowermost line 43. As pointed our more fully hereinafter, a predetermined profile for the effort to be exerted by the exercising person and the pacing of that effort may be coordinated with signalled force and position so that, when the programmed regimen is followed, the user responsive trace or gradually extending line tracks constantly along a single predetermined line such as the line 44 in coordination with the pace of the progressive pacing signal extending along the line 41. With an apparatus such as the leg extension apparatus 20 described above, indicator signals to either side of the display area occupied by the lines 41 and 43 through 46 may be used to indicate which limb or body portion is to be engaged in doing positive work at any given moment.

An exercise regimen designed for use with an apparatus such as the leg extension machine 20 typically will involve a predetermined number of repetitions of cycles of movement. As repetitions occur, a digital display presents numerical indicia of the number of repetitions which have been completed. At the conclusion of the number of repetitions programmed by the regimen, then the average force exerted and the average interval of time required for a repetition may be displayed as numerical indicia and combined into a score.

Where a plotting attachment or a printer is associated with the central processing unit, the information thus derived may also be presented in graphic form. One form of graphic representation is illustrated in Figure 5, where curves have been plotted as the average of repetitions by the left leg and repetitions by the right leg for an exercising person using a leg extension machine.

The present invention contemplates that operation of exercise machines in accordance with a stored program may involve operation of the apparatus with the microprocessor unit (MPU) which forms a portion of an individual apparatus or by cooperation between the MPU and a central processor unit (CPU). In instances where operation is essentially under the control of an MPU, instructions regarding the standard magnitude of force to be exerted by the exercising person and the standard rate of exertion of force by the exercising person and the standard number of repetitions of cyclical force exertion will be entered directly at the MPU, either manually by the exercising person prior to beginning use of the apparatus 20 or from a stored record card such as a punched card or a magnetic record bearing card which may be inserted into a reader at the apparatus 20. Where operation is in coordination with a CPU, the greater information storage capabilities of the CPU may permit operation in accordance with a procedure wherein an individual merely identifies themselves to the CPU and the CPU thereupon loads into the MPU the instructions required.

The components involved in operation as described hereinabove may be as schematically represented in Figure 6, where the analogue signalling devices incorporated into an exercising apparatus are indicated, as are the MPU, display and CPU.

In circumstances where a CPU is used in association with exercise machines, the "intelligent machine" characteristic of exercise apparatus in accordance with the present invention permits a single CPU to be shared among and communicate with a plurality of individual exercise machines. This is significant in that up to at least thirty-two (32) distinct types of exercise machines are contemplated as being useful along the general lines described hereinabove with specific reference to the leg extension machine 20. Thus, a single CPU may monitor, process and display information derived from a plurality of exercise apparatus. The CPU may also direct the full exercise regimen of an individual using the facility so equipped.

A simplified flow chart of the cooperation between a CPU and an MPU is represented in Figure 7. As will be noted, information is transferred between the MPU and CPU upon completion of each body portion movement in one particular direction. That is, a "repetition" is considered to be a full cycle of force exertion. A "half repetition" represents one-half cycle of a cycle of force exertion, during which one body portion is doing positive work and the other body portion is doing negative work, in the instance of the leg extension apparatus 20.

As mentioned briefly above, it is contemplated that the present invention has utility with a number of exercise apparatus other than the leg extension apparatus 20 described hereinabove. For purpose of illustrating a different type of apparatus, Figures 8 through 12 have been included as showing a type of apparatus known as a hip-back apparatus. In using the hip and back apparatus generally indicated at 60, an exercising person assumes a supine position with the hip joints aligned with the axis of rotation of a pair of roll members 61, 62. The hip and back exercise apparatus 60 includes, as shown in Figures 8 through 10, a display generally indicated at 40 identical with the display used with the leg extension machine shown in Figures 1 through 3.

The hip and back apparatus 60 is constructed and operates in a manner generally comparable to the leg extension apparatus 20 of Figures 1 through 3 as described more fully hereinabove. The hip and back apparatus 60 includes a chain 71 trained about a pair of sprockets 68, 69 so that arms 64, 65 mounting the roll members 61, 62 move in opposite directions with coordinated movement. A load cell 70 is incorporated in the chain.

The hip and back apparatus 20 makes more clear the use of apparatus in accordance with the present invention as a force measuring apparatus or tensiometer. More particularly, first and second locking levers 74, 75 are provided and are actuable by a user of the machine to lock respective ones of the sprockets 68, 69 against rotation. As made more clear by Figures 11 and 12, such locking of a sprocket 69 against rotation occurs by insertion of a tooth 78 into the teeth of the sprocket.

In using an apparatus in accordance with the present invention as a force measuring apparatus or tensiometer, a respective one of the arms 64, 65 is brought to a position in which a forced measurement is to be made and the sprocket at the opposite side of the machine is locked by operating the appropriate lever. Thus, if the force exerted by a left leg is to be measured by determining the force exerted against one roll member 61, the locking lever 74 on the opposite side of the apparatus 60 is actuated to lock the remote sprocket 68. Upon this occurrence, the load cell 70 is located in the "tight side" of the closed loop of the chain 71. Thus, force exerted by a user of the apparatus 60 would be developed statically, with the body portion through which force was being exerted blocked against movement in the direction that force was being exerted, and force measurements would be available from a range of positions.

As will be appreciated, use of exercise apparatus as described hereinabove with particular reference to two specific forms opens possibilities for accurate determination of effort expended during an exercise regimen. By the same token, goals for an exercise regimen may be established from a basis of knowledge concerning an individual's abilities to sustain an extended effort and a desired goal for such an ability. Finally, where an individual's starting ability and a desired goal can both be adequately represented from information monitored and processed by the apparatus as described above, then the exercise activity of a user may be directed by means of the display 40 so as to optimize achievement of the established goals.

## Claims

1. Exercise apparatus (20, 60) for providing a user with a range of exercise movements and recording a measure of the effort exerted, said apparatus including;

first body engaging means (21, 61) adapted to be contacted by a first portion of a user's body and to be moved therewith;

second body engaging means (22, 62) adapted to be contacted by a second portion of a user's body and to be moved therewith;

interconnecting means (31, 71) linking said first and second body engaging means in a force transmitting passive relationship for effecting predetermined coordinated movement of said first and second body engaging means with respect to one another, said interconnecting means converting positive musculature work applied to one of said body engaging means to negative musculature work supplied to other body engaging means;

force sensing means (70) operatively connected to said interconnecting means and producing an output signal proportional to the magnitude of the force applied to at least one of said body engaging means; and

display means (40) responsive to said output signal to generate a cognizable display;

characterized by:

means whereby all forces and resistances are provided solely by the user and may be instantaneously varied by said user such that there are substantially no other forces applied to and no resistive forces acting upon said body engaging means (21, 22; 61, 62);

position sensing means operatively connected to said interconnecting means (31, 71) and producing an output signal proportional to the position of at least one of said body engaging means; and

processing means responsive to said force sensing means (70) and to said position sensing means to provide an output signal proportional to the physical effort exerted by the user as represented by the applied forces and changes of position.

2. Apparatus according to claim 1, further having selectively programmable standard producing means to provide an output signal proportional to the desired amount of physical effort to be exerted by an exercising person, said display means being responsive to said processing means output signal and said standard producing output signal to generate a cognizable display.

3. Apparatus according to claim 1 or 2, wherein said interconnecting means includes rotational means connected to said first and second body engaging means, said rotational means having an axis of rotation coaxial with an axis of rotation of said contacted portion of said user's body to constrain movement of said first and second body engaging means to an arcuate movement corresponding to the natural arcuate movement of said contacted portion of said user's body.

4. Apparatus according to claim 3, wherein said rotational means is coaxial with a user's body joint.

5. Apparatus according to any preceding claim, wherein said interconnecting means includes non-extensible means connecting said first and second body engaging means.

6. Apparatus according to any preceding claim, wherein said interconnecting means includes a first rotational means operatively connected to said first body engaging means for rotation therewith, a second rotational means operatively connected to said second body engaging means for rotation therewith, and connecting means operatively connecting said first and second rotational means so that rotation of one rotational means results in rotation of said other rotational means.

7. Apparatus according to any preceding claim, wherein movement of said first body engaging means in one direction results in movement of said second body engaging means in an opposite direction.

8. Apparatus according to any preceding claim, wherein said force sensing means includes an electromechanical strain gauge that provides an electrical output signal.

9. Apparatus according to any preceding claim, wherein said position sensing means produces an electrical output signal.

10. Apparatus according to any preceding claim, wherein said processing means includes a programmable microprocessor.

11. Apparatus according to any preceding claim, wherein said display means comprises a plurality of light emitting diodes in a predetermined array, each light emitting diode being responsive to a predetermined signal from said processing means.

12. Apparatus according to any preceding claim, wherein said display means comprises a cathode ray tube means.

**Revendications**

1. Dispositif d'exercice (20, 60) pour mettre à la disposition d'un utilisateur une gamme de mouvements d'exercice et enregistrer une mesure de l'effort exercé, ledit dispositif comprenant:

— un premier moyen d'engagement (21, 61) du corps adapté pour être relié avec une première partie du corps d'un utilisateur et pour être mis en mouvement avec;

— un deuxième moyen d'engagement (22, 62) adapté pour être relié avec une deuxième partie du corps d'un utilisateur et pour être mis en mouvement avec;

— un moyen d'interconnexion (31, 71) reliant lesdits premier et deuxième moyens d'engagement en relation passive de transmission de force pour l'accomplissement d'un mouvement coordonné prédéterminé desdits premier et second moyens d'engagement du corps l'un par rapport à l'autre, ledit moyen d'interconnexion transformant un travail musculaire positif appliqué à l'un desdits moyens d'engagement du corps en un travail musculaire négatif transmis à l'autre moyen d'engagement du corps;

— un moyen de détection des forces, fonctionnellement relié audit moyen d'interconnexion et produisant un signal de sortie proportionnel à l'intensité de la force appliquée à au moins un desdits moyens d'engagement du corps, et,

— un moyen d'affichage sensible audit signal de sortie pour générer un affichage reconnaissable;

caractérisé en ce qu'il comporte en outre:

— des moyens par lesquels toutes les forces et résistances sont fournies seulement par l'utilisateur et peuvent être instantanément changées par ledit utilisateur tels qu'il n'y a réellement pas d'autres forces appliquées à, ni de forces résistances agissant sur, lesdits moyens d'engagement du corps (21, 22; 61, 62);

— un moyen de détection de position fonctionnellement relié audit moyen d'interconnexion (31, 71) et produisant un signal de sortie proportionnel à la position d'au moins un desdits moyens d'engagement du corps, et,

— un moyen de traitement sensible audit moyen de détection des forces (70) et audit moyen de détection de position pour fournir un signal de sortie proportionnel à l'effort physique exercé par l'utilisateur tel qu'il se manifeste par les forces appliquées et les variations de position.

2. Dispositif selon la revendication 1, ayant de plus des moyens de production courants sélectivement programmables pour fournir un signal de sortie proportionnel à la quantité souhaitée d'effort physique à exercer par la personne s'exerçant, ledit moyen d'affichage étant sensible au signal de sortie dudit moyen de traitement et au signal de sortie desdits moyens de production courants pour générer un affichage reconnaissable.

3. Dispositif selon l'une des revendications 1 et 2, caractérisé en ce que ledit moyen d'interconnexion comprend un moyen de rotation connecté auxdits premier et deuxième moyens d'engagement du corps, ledit moyen de rotation ayant un axe de rotation coaxial avec un axe de rotation de ladite partie du corps reliée dudit utilisateur pour contraindre le mouvement desdits premier et deuxième moyens d'engagement du corps à un mouvement en forme d'arc correspondant au mouvement naturel en forme d'arc de ladite partie reliée du corps dudit utilisateur.

4. Dispositif selon la revendication 3, caractérisé en ce que ledit moyen de rotation est coaxial avec une articulation du corps de l'utilisateur.

5. Dispositif selon l'une quelconque des revendications précédentes, caractérisé en ce que ledit moyen d'interconnexion comprend des moyens inextensibles reliant lesdits premier et deuxième moyens d'engagement du corps.

6. Dispositif selon l'une quelconque des revendications précédentes, caractérisé en ce que ledit moyen d'interconnexion comprend un premier moyen de rotation fonctionnellement relié audit premier moyen d'engagement du corps pour sa rotation, un deuxième moyen de rotation fonctionnellement relié audit deuxième d'engagement du corps pour sa rotation, et des moyens de connexion reliant fonctionnellement lesdits premier et deuxième moyens de rotation de façon à ce que la rotation de l'un des moyens de rotation entraîne la rotation de l'autre moyen de rotation.

7. Dispositif selon l'une quelconque des revendications précédentes, caractérisé en ce que le mouvement dudit premier moyen d'engagement du corps dans une direction entraîne le mouvement dudit deuxième moyen d'engagement du corps dans une direction opposée.

8. Dispositif selon l'une quelconque des revendications précédentes, caractérisé en ce que ledit moyen de détection des forces comprend une jauge de contrainte électromécanique qui fournit un signal de sortie électrique.

9. Dispositif selon l'une quelconque des revendications précédentes, caractérisé en ce que ledit moyen de détection de position produit un signal de sortie électrique.

10. Dispositif selon l'une quelconque des revendications précédentes, caractérisé en ce que ledit moyen de traitement comprend un microprocesseur programmable.

11. Dispositif selon l'une quelconque des revendications précédentes, caractérisé en ce que ledit moyen d'affichage comprend une pluralité de diodes électroluminescentes dans un arrangement prédéterminé, chaque diode électroluminescente étant sensible à un signal prédéterminé depuis ledit moyen de traitement.

12. Dispositif selon l'une quelconque des revendications précédentes, caractérisé en ce que ledit moyen d'affichage comprend un moyen de tube à rayons cathodiques.

**Patentansprüche**

1. Übungsvorrichtung (20, 60), durch die ein Benutzer eine Reihe von Übungsbewegungen ausüben kann und durch die eine Messung der ausgeübten Kraft aufgezeichnet wird, bestehend aus:

einem ersten, an den Körper angreifenden Mittel (21, 61), welches geeignet ist, von einem ersten Teil des Benutzers berührt und hierdurch bewegt zu werden;

zweiten, an den Körper angreifenden Mitteln (22, 62), die geeignet sind, von einem zweiten Teil des Benutzerkörpers berührt und damit bewegt zu werden;

Verbindungsmitteln (31, 71), die die ersten und zweiten angreifenden Mittel in einem eine Kraft übertragenden, passiven Verhältnis gelenkig verbinden, um eine bestimmte koordinierte Bewegung der ersten und zweiten Mittel zueinander zu bewirken, wobei die Verbindungsmittel positive Muskelarbeit, die dem einen Mittel zugeführt wird, in negative Muskelarbeit, die dem anderen Mittel zugeführt wird, umformen;

die Kraft abtastende Mittel (70), die wirksam mit den erwähnten Verbindungsmitteln verbunden sind und ein Ausgangssignal proportional der Größe der Kraft erzeugen, die mindestens einem der den Körper berührenden Mittel zugeführt wird und

Darstellungsmittel (40), die auf das erwähnte Ausgangssignal ansprechen und ein erkennbares Bild erzeugen;

gekennzeichnet durch:

Mittel, durch die alle Kräfte und Widerstände allein durch den Benutzer erzeugt werden und sofort durch den Benutzer so verändert werden können, daß im wesentlichen keine anderen Kräfte vorhanden sind, die den erwähnten, den Körper berührenden Mitteln zugeführt werden und keine Widerstandskräfte vorhanden sind, die auf die den Körper berührenden Mittel einwirken (21, 22, 61, 62);

das Einstellen der Abtastmittel, die wirksam mit den Verbindungsmitteln (31, 71) verbunden sind und die ein Ausgangssignal proportional zu der Einstellung mindestens eines der den Körper berührenden Mittel erzeugen und

Verarbeitungsmittel, die auf die erwähnten, die Kraft abtastenden Mittel (70) und auf die Einstellung der Abtastmittel ansprechen, um ein Ausgangssignal proportional der physikalischen Kraft zu erzeugen, die durch den Benutzer ausgeübt wird, wie durch die zugeführten Kräfte und Änderungen der Einstellung dargestellt ist.

2. Vorrichtung nach Anspruch 1, dadurch gekennzeichnet, daß weiter wahlweise programmierbare Standardherstellungsmittel vorgesehen sind, um ein Ausgangssignal proportional der gewünschten Größe der durch eine übende Person auszuübenden physikalischen Kraft vorzusehen, wobei die Bildmittel auf das Ausgangssignal der Verarbeitungsmittel und auf das üblich erzeugte Ausgangssignal ansprechen, um ein erkennbares Bild zu erzeugen.

3. Vorrichtung nach Anspruch 1 oder 2, dadurch gekennzeichnet, daß die Verbindungsmittel Rotationsmittel aufweisen, die mit den ersten und zweiten, an den Körper angreifenden Mitteln verbunden sind, wobei die Rotationsmittel eine Drehachse koaxial zur Drehachse des berührten Teiles des Benutzerkörpers ist, um die Bewegung der ersten und der zweiten, an den Körper zur Anlage kommenden Mittel in eine Bogenbewegung zu zwingen entsprechend der natürlichen Bewegung des berührten Teiles des Benutzerkörpers.

4. Vorrichtung nach Anspruch 3, dadurch gekennzeichnet, daß die Rotationsmittel koaxial zum Gelenk eines Benutzerkörpers sind.

5. Vorrichtung nach einem der Ansprüche 1 bis 4, dadurch gekennzeichnet, daß die Verbindungsmittel nicht dehnbare Mittel aufweisen, die die ersten und die zweiten an den Körper zur Anlage kommenden Mittel verbinden.

6. Vorrichtung nach einem der Ansprüche 1 bis 5, dadurch gekennzeichnet, daß die Verbindungsmittel erste Rotationsmittel aufweisen, die wirksam mit den ersten, an den Körper anliegenden Mitteln zur Verdrehung verbunden sind, daß ein zweites Rotationsmittel wirksam mit dem zweiten, an den Körper zur Anlage kommenden Mitteln verbunden ist und daß verbindende Mittel wirksam das erste und zweite Rotationsmittel derart verbinden, daß die Drehung des einen Rotationsmittels zur Drehung des anderen Rotationsmittels führt.

7. Vorrichtung nach einem der Ansprüche 1 bis 6, dadurch gekennzeichnet, daß die Bewegung des ersten, an den Körper zur Anlage kommenden Mittels in eine Richtung zu einer Bewegung des zweiten Mittels in entgegengesetzte Richtung führt.

8. Vorrichtung nach einem der Ansprüche 1 bis 7, dadurch gekennzeichnet, daß die die Kraft abtastenden Mittel ein elektromechanisches Belastungs-Meßgerät aufweisen, welches ein elektrisches Ausgangssignal erzeugt.

9. Vorrichtung nach einem der Ansprüche 1 bis 8, dadurch gekennzeichnet, daß die die Einstellung abtastenden Mittel ein elektrisches Ausgangssignal erzeugen.

10. Vorrichtung nach einem der Ansprüche 1 bis 9, dadurch gekennzeichnet, daß die Verarbeitungsmittel einen programmierbaren Mikroprozessor aufweisen.

11. Vorrichtung nach einem der Ansprüche 1 bis 10, dadurch gekennzeichnet, daß die Darstellungsmittel eine Anzahl von Licht emittierenden Dioden in bestimmter Reihe besitzen, wobei jede Licht emittierende Diode auf ein bestimmtes Signal aus den Verarbeitungsmitteln anspricht.

12. Vorrichtung nach einem der Ansprüche 1 bis 11, dadurch gekennzeichnet, daß die Darstellungsmittel eine Kathodenstrahlröhre besitzen.

0 057 609

FIG-1

FIG-2

FIG-3

1

Fig-4

FORCE

FORCE VS. POSITION TEST

CURVE L: AVG.OF LEFT REPS.
CURVE R: AVG.OF RIGHT REPS.
TEST: 75  10/29/80  13:55 EXERCISE: LEG EXTENSION
REPS: 10  FORCE: 10A

SCORES   FORCE: 39  TIME: 40   TOTAL: 79

Fig-5

CHECK STATUS

USER REQUEST — N

Y

REQUEST TO CPU

IS CPU OPERATIVE — N → REQUEST USER ENTER REQUIREMENTS

Y

LOAD USER TABLES

DISPLAY REPETITIONS TO BE DONE AND COUNTDOWN

DO HALF REPETITION

IS CPU OPERATIVE — N

Y

SEND HALF REPETITION RECORD

LAST HALF REPETITION — N

Y

COMPUTE SCORES

IS CPU OPERATIVE — N

Y

SEND SCORES AND STATUS

DISPLAY SCORES

CPU

MPU

30

29

31

34

34

70

40

_Fig-6_

_Fig-7_

Fig-8

Fig-9

Fig-10

Fig-11

Fig-12